# EUROPEAN PATENT APPLICATION

(11) **EP 3 000 337 A1**
(43) Date of publication of application: **30.03.2016**
(21) Application number: 13770619.8
(22) Date of filing: 20.06.2013
(51) Int. Cl.: A24F 47/00

(54) **NEW PLANT ESSENTIAL OIL NEBULIZER**

(30) Priority: 21.05.2013 CN 201320287400 U
(71) Applicant: Shenzhen Boge Technology Co., Ltd, Shenzhen, Guangdong 518000 (CN)
(72) Inventor: ZHENG, Junxiang, Shantou Guangdong 515057 (CN); ZHANG, Xianhui, Shenzhen Guangdong 518000 (CN)
(74) Representative: Chaillot, Geneviève
(86) International application number: PCT/CN2013/077525
(87) International publication number: WO 2014/186996

(57) **Abstract**

A new kind of plant essential oil vaporizer comprising a vaporizer main body, a vaporizer socket disposed on the vaporizer main body and a pair of electrode poles disposed inside the vaporizer socket; two openings are disposed on the electrode poles respectively; a heating wire is disposed above the electrode poles and they are connected via rivets and the openings. The new kind of plant essential oil vaporizer has the following advantages: the electrode poles and the heating wire are connected directly by riveting, therefore assisting conduction wire and riveting copper columns are not required; the electrode poles constitute two electrical poles of the vaporizer inside the vaporizer socket. The vaporizer has a simple structure and low manufacturing cost, and only requires simple manufacturing technique and can be manufactured efficiently.

## Description

### Technical Field

The present invention relates to a new kind of plant essential oil vaporizer.

### Background Art

Nowadays, the use of plant essential oil vaporizer in lieu of a real cigarette is environmentally friendly and free from the hazards caused by second hand smoking and is therefore well-received in the market. A plant essential oil vaporizer contains a heating wire inside. To operate the vaporizer, the heating wire is required to be welded with the two electrical poles of the vaporizer via a conduction wire to achieve conduction, and then the two electrical poles of the vaporizer are required to contact the cathode and anode of the power supply respectively to achieve conduction.

Nowadays, the two electrical poles of the vaporizer is formed by an elongated metal ring and an outer screw thread ring with an insulation ring disposed in between the elongated metal ring and the outer screw thread ring to connect the elongated metal ring and the outer screw thread ring. Two ends of the heating wire are connected with the conduction wire via riveting copper columns. Two ends of the conduction wire are welded on the elongated metal ring and the outer screw thread ring respectively.

The vaporizer as described above requires riveting between the heating wire and the conduction wire during production; after riveting, the heating wire is required to be welded with the two electrical poles of the vaporizer; also, structural parts of the two electrical poles of the vaporizer require to be manufactured by using sophisticated lathe. In view of the above, production of this kind of vaporizer is complicated and the cost of production is very high.

### Disclosure of the Invention

In view of the aforesaid disadvantages now present in the prior art, the present invention provides a new kind of plant essential oil vaporizer which has simple structure and good sealing effect.

The present invention has the following technical proposal:
A new kind of plant essential oil vaporizer comprising a vaporizer main body, a vaporizer socket disposed on the vaporizer main body and a pair of electrode poles disposed inside the vaporizer socket; two openings are disposed on the electrode poles respectively; a heating wire is disposed above the electrode poles and they are connected via rivets and the openings.

As a preferred embodiment, the vaporizer socket is disposed with a sealing stopper.

As a preferred embodiment, the electrode poles are different in their polarities.

The present invention has the following advantages by adopting the above technical proposal: The electrode poles and the heating wire are connected directly by riveting, therefore assisting conduction wire and riveting copper columns are not required; the electrode poles constitute two electrical poles of the vaporizer inside the vaporizer socket. The present invention has a simple structure and only requires simple manufacturing technique; the present invention can be manufactured efficiently and the manufacturing cost is low.

### Brief Description of Drawings

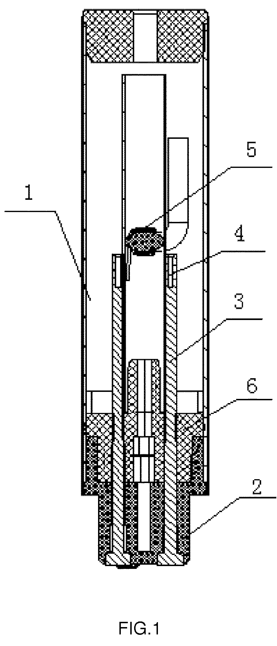

FIG. 1 is a structural illustration of the present invention.

### Reference numbers in the drawings:

1: Vaporizer main body; 2: Vaporizer socket; 3: electrode poles; 4: openings; 5: heating wire; 6: sealing stopper.

### Best Mode for Carrying out the Invention

The present invention is further described in detail below with reference to the accompanying drawings.

As shown in FIG. 1, a new kind of plant essential oil vaporizer according to the present invention comprises a vaporizer main body 1, a vaporizer socket 2 disposed on the vaporizer main body 1 and a pair of electrode poles 3 disposed inside the vaporizer socket 2; two openings 4 are disposed on the electrode poles 3 respectively; a heating wire 5 is disposed above the electrode poles 3 and they are connected via rivets and the openings 4.

The vaporizer socket 2 is disposed with a sealing stopper 6. The sealing stopper 6 prevents plant essential oil inside a vaporizer tube from leaking out from the vaporizer.

As a preferred embodiment, the electrode poles 3 are different in their polarities.

Operation principle: the electrode poles 3 and the heating wire 5 are connected directly by riveting, therefore assisting conduction wire and riveting copper columns are not required; the electrode poles 3 constitute two electrical poles of the vaporizer inside the vaporizer socket 2.

The present invention has the following advantages: The present invention has a simple structure and only requires simple manufacturing technique; the present invention can be manufactured efficiently and the manufacturing cost is low.

The above description is only a preferred embodiment of the present invention. Any changes and modification of the present invention in accordance with the structure, characteristics and principles defined by the scope of the present invention should fall within the scope of protection of the present invention.

## Claims

1. A new kind of plant essential oil vaporizer comprising a vaporizer main body (1), a vaporizer socket (2) disposed on the vaporizer main body (1) and a pair of electrode poles (3) disposed inside the vaporizer socket (2); two openings (4) are disposed on the electrode poles (3) respectively; a heating wire (5) is disposed above the electrode poles (3) and they are connected via rivets and the openings (4).

2. The new kind of plant essential oil vaporizer as in Claim 1, wherein the vaporizer socket (2) is disposed with a sealing stopper (6).

3. The new kind of plant essential oil vaporizer as in Claim 2, wherein the electrode poles (3) are different in their polarities.
